# EUROPEAN PATENT APPLICATION

(11) **EP 1 666 019 A1**
(43) Date of publication of application: **07.06.2006**
(21) Application number: 04772654.2
(22) Date of filing: 26.08.2004
(51) Int. Cl.: A61K 6/00, A61K 8/18, A61C 13/00, C09J 193/02, C09J 193/04, A61P 1/02

(54) **COMPOSITION FOR APPLICATION TO TOOTH**

(30) Priority: 27.08.2003 JP 2003302282
(71) Applicant: HANIX CO., LTD., Tokyo 124-0022 (JP)
(72) Inventor: URAI, Kaoruko, Katsushika-ku, Tokyo 124-0022 (JP); TODOROKI, Reiko, Narashino-shi, Chiba 275-0012 (JP)
(74) Representative: Dokter, Eric-Michael
(86) International application number: PCT/JP2004/012698
(87) International publication number: WO 2005/020937

(57) **Abstract**

The invention provides a tooth coating composition for beauty and appearance and prevention of dental caries and excellent in the quick drying property, durability, color tone, luster, and removal easiness by using shellac and rosin as main components and a pigment, a pigment dispersant, a dye, an efficacious component having anti-dental caries function, an anti-bacterial agent, a tackifier, a luster reflectingmaterial, a bleaching agent, and a flavor as auxiliary components.

## Description

### BACKGROUND OF THE INVENTION

### Field of the Invention

The invention relates to a tooth coating composition usable for a tooth coating agent to be applied to teeth for increasing the effects on dental health, or beauty and appearance, or for an adhesive for sticking a seal, crushed stone, a luster reflecting agent for purposes of arts, or for a tooth paint for coloring teeth for purposes of arts.

### Description of the Related Art

So far, some tooth coating compositions for the purposes of improving the beauty and the health of teeth have been developed and commercialized. These tooth coating compositions are required to have the performance such as the safety for the human body, the dispersibility of pigments, the hiding power for covering the blackish portions of teeth, the aesthetic property of the luster of the coat, being hard to cause changes with the passage of time, and the easiness of removal of the coat, etc.

Recently,tooth coating compositions having a quick drying property to complete coating within shorter time and improved durability of an applied coat have been proposed.

In Japanese Patent Application Laid-Open No. 2002-187809, there is disclosed a tooth coating composition increasing the adhesion property of shellac and excellent in the quick drying property and durability of an applied coat by using the shellac in combination with mica titanium, however it is not yet sufficiently satisfactory in the hiding power and the luster j ust like natural teeth. Further, in the case of a liquid product with decreased viscosity, the product has a defect that it drips during application and is difficult to be applied to the teeth in the lower jaw.

If the ratio of the pigment is increased to increase the hiding power, the durability of the applied coat is shortened and the coating unevenness easily occurs, resulting in result in a problem of deterioration of the aesthetic property. If the concentration of the shellac to prolong the durability of the applied coat is increased, it takes a long time for drying to result in difficulty for use and the coat becomes opaque with the passage of time to result in a problem of deterioration of the aesthetic property.

### SUMMARY OF THE INVENTION

The invention aims to provide a tooth coating composition to be applied to form a coat giving luster similar to that of natural teeth, durability of the applied coat, excellence of hiding power, and high safety to human body in the case it is applied to teeth (natural teeth, artificial teeth, or artificial crowns of teeth) by using shellac and rosin in combination. Moreover, without losing the quick drying property, the resin concentration can be increased by well combining the different properties of the shellac and rosin resins and accordingly, the strength of the coat can be heightened and the durability of the coat applied to teeth can be expanded.

The tooth coating composition is provided by adding one or more kinds of auxiliary components selected from a pigment, a pigment dispersant, a dye, an efficacious component having anti-dental caries function, an anti-bacterial agent, a tackifier, a luster reflecting material, a bleaching agent, and a flavor to a composition containing shellac and rosin as main components and is usable for various applications for a tooth coating agent for beauty and appearance, a tooth coating agent for preventing teeth from dental caries, an adhesive for art materials for teeth, a painting agent for teeth.

The rosin to be used in the invention is a solid resin obtained by removing essential oil from extract of Pinus plants and to be used while being crushed finely and dissolved in ethanol. The content of the rosin to be added to a shellac ethanol solution in the entire composition is preferably in a range of 1 wt.% to 30 wt. %. If it is less than 1 wt. %, the effect of strengthening a coat is not so sufficient and if it exceeds 30 wt.%, the composition becomes difficult to be used as a tooth coating agent due to difficulty in drying it. The content of the rosin in the entire composition is more preferably in a range of 5 wt.% to 25 wt. %. The rosin contains a large quantity of abietic acid. The abietic acid has antibacterial function to Streptococcus mutans, which is bacterium causing dental caries. Accordingly, use of the rosin is effective for preventing tooth decay without addition of any pharmaceutically efficacious component having the above-mentioned anti-dental caries function.

The content of the shellac in the entire composition is preferably in a range of 1 wt.% to 30 wt.%. If it is less than 1 wt.%, the adhesion property to teeth becomes weak to make the composition unsuitable for practical use as a coating agent. If it is more than 30 wt.%, the composition becomes difficult to be dries and easy to be opaque, resulting in deterioration of the aesthetic property. The content of the shellac in the entire composition is more particularly preferable to be in a range of 5 wt.% to 20 wt.%.
The total content of the shellac and rosin in the entire composition is preferably in a range of 10 wt.% to 31 wt.%. If the total content of both is less than 10 wt.%, the strength of the coat is decreased and the hiding power of the color of teeth thereunder is decreased and therefore, no beautiful coat is obtained. On the other hand, if the total content of both is more than 31 wt.%, the quick drying property is deteriorated and the viscosity is too high to apply, resulting in easy formation of an uneven coat.

Generally, the shellac is commercialized in form of an ethanol solution dissolved therein. Laccoat 50 EDS (50% shellac ethanol solution: manufactured by Japan Shellac Industries, Ltd.) is made available as a commercialized product.

As the pigment may be used titanium oxide, mica titanium, natural pearl, iron oxide, a body extender pigment, and a synthetic coloring agent. The addition ratio of the pigment is preferably in a range of 1 wt.% to 20 wt.%.

As the pigment dispersant may be used an N-methacrylethyl-N,N-dimethylammoniul-α-N-methylcarboxybeta ine-alkyl methacrylate copolymer. Yukaformer R 202 (manufactured by Mitsubishi Yuka Fine Chemicals Co., Ltd.) commercialized in form of an ethanol solution containing 30 wt. % of the above-mentioned copolymer is preferable to be used.

The content of the above-mentioned N-methacrylethyl-N,N-dimethylammonium-α-N-methylcarboxybeta ine-alkyl methacrylate copolymer in the entire composition is preferably in a range of 1 wt.% to 20 wt.%.

A various kinds of dyes allowed for use as food additives may be used for the dye. Addition of a dye or a synthetic coloring element to the tooth coating composition of the invention makes the composition usable for a tooth paint for designing various patterns to teeth.

As the pharmaceutically efficacious component having the anti-dental caries function, one or more of compounds selected from sodium fluoride, sodium monofluorophosphate, and stannous fluoride may be added.

As the anti-bacterial agent may be used sodium azulenesulfonate, ε-aminocaproic acid, allantoin, allantoin chlorohydroxyaluminum, allantoin dihydroxyaluminum, epidihydrocholesterol, dihydrocholesterol, sodium chloride, glycyrrhizic acid, diammonium glycyrrhizinate, disodium glycyrrhizinate, trisodium glycyrrhizinate, dipotassium glycyrrhizinate, monoammonium glycyrrhizinate, β-glycyrrhetic acid, isopropylmethylphenol, cetylpyridinium chloride, decalinium chloride, benzalconium chloride, benzethonium chloride, alkyldiaminoethylglycine hydrochloride, chlorohexidine hydrochloride, triclosan, ascorbic acid, sodium ascorbate, pyridoxine hydrochloride, dl-α-tocopherol acetate, dl-α-tocopherol nicotinate, zeolite, sodium dihydrogen pyrophosphate, sodium pyrophosphate, sodium monohydrogen phosphate, trisodium phosphate, sodium polyphosphate, polyethylene glycol, polyvinyl pyrrolidone, lysozyme chloride, sodium copper chlorophyllin, hinokitiol, polyoxyethylene lauryl ether, sodium lauroyl sarcosinate, plant extract, and phytoncide.

As the plant extract may be used fig extract, hydrangea tea extract, oolong tea extract, green tea extract, grape seed extract, grape skin extract, blueberry extract, apple extract, eucalyptus extract, Rome chamomile extract, rosemary extract, fennel extract, myrrh extract, lemon extract, peppermint extract, and sage extract. These extracts have a deodorization effect other than the anti-bacterial function.

Phytoncide is an essential oil obtained by extracting the sap obtained from leaves and trunks of a tree by distillation or the like and has a deodorization function other than the anti-bacterial function.

As the tackifier, hydroxypropyl cellulose (HPC) may be added. Addition of the tackifier increases the viscosity of the tooth coating composition and provides the tooth coating composition whose dripping is prevented in the case of application to teeth in the lower jaw and with which coating can be carried out excellently.
As the bleaching agent may be used hydrogen peroxide, calcium peroxide, and urea peroxide.

As the art material may be used sheets made of paper or plastics and having a variety of shapes such as a star-like, heart-like, or rhombic shape, fine granules and fine powder obtained by milling stones with beautiful colors, and fine powders and fine fragments of a luster reflecting material, so-called lame. These art materials may be used for sticking or scattering them when the tooth coating composition is still in half-dry state after the tooth coating composition of the invention is applied to the tooth surface so as to draw a design and heighten the aesthetic property of teeth.

As the luster reflecting material may be used a poly(ethylene terephthalate)-aluminum-epoxy laminate, a poly(ethylene terephthalate)-metal laminate, a poly(ethylene terephthalate)-polyolefin laminate, a poly(ethylene terephthalate)-poly(methyl methacrylate) laminate, a polyethylene-poly(ethylene terephthalate) laminate, and a polyethylene-terephthalic acid-pentaerythritol type polyester laminate. These luster reflecting materials may be added directly to the tooth coating composition and may be stuck to teeth using the composition as an adhesive.
The effects of the invention are as follows.

(1) The invention provides a tooth coating composition excellent in luster just like natural teeth, the hiding power, the durability of an applied coat, and easiness of removal of a used coat, and is safe for human body by mixing shellac 1 wt.% to 30 wt.% and rosin 1 wt.% to 30 wt.%.

(2) Addition of a pigment 1 wt.% to 20 wt.%, the color tone can be adjusted. The pigment is preferable to be added in an amount of 5 wt.% or more so as to cover blackish tooth color. Also, addition of a dye makes the composition usable as a paint for tooth art.

(3) Addition of N-methacrylethyl-N,N-dimethylammonium-α-N-methylcarboxybeta ine-alkyl methacrylate copolymer as a dispersant of a pigment makes the dispersibility of a pigment good and prevents uneven dispersion and increases the viscosity, resulting in easiness of application. Also, the addition provides the tooth coating composition with luster and transparent impression.

(4) Addition of an anti-bacterial agent such as sodium fluoride, sodium monofluorophosphate, and stannous fluoride promotes the preventive effect on tooth decay. Also the concentration of the resin can be increased and the thickness of the applied coat can be thick and owing to a sealant effect, the tooth decay-preventive effect can synergistically improved.

(5) Addition of hydrogen peroxide, calcium peroxide, urea peroxide, or the like as a bleaching agent makes it possible to decompose the coloring elements of discolored teeth by applying the composition to the teeth and further whiten the teeth.
(6) The composition may be used as an adhesive for sticking an art material such as a seal, a crushed stone, and a luster reflecting material to teeth. In this case, since the composition has luster just like natural teeth with little alteration with the passage of time, the composition scarcely deteriorates the aesthetic property of the art designed on teeth.

### DESCRIPTION OF THE PREFERRED EMBODIMENTS

Compositions obtained by adding a variety of auxiliary components to combinations of shellac 10 wt.% and rosin in a blending ratio changed in a range of 5 wt.% to 20 wt.% and compositions for comparison containing neither shellac nor rosin were subjected to comparative tests of the quick drying property, the luster, the durability of applied coats, and removal easiness of used coats.

### [Example 1]

Rosin 15.0 wt.%, Laccoat 50 EDS (50% shellac ethanol solution) 20.0 wt.% (concentration of shellac alone is 10 wt.%), absolute ethanol 36.7 wt.%, titanium oxide-Yukaformer ethanol solution (produced by dispersing silicic acid-treated titanium oxide 10.0 wt.% in a solution containing Yukaformer R 202 66.7 wt.% and absolute ethanol 23.3 wt.%) 25.0 wt.%, a flavor 2.30 wt.%, and rosemary extract 1.00 wt.% were well mixed by a stirrer to obtain an aimed composition. The ratio of titanium oxide to the entire composition is 2.5 wt.%.

### [Example 2]

Rosin 15.0 wt.%, Laccoat 50 EDS 20.0 wt.%, absolute ethanol 54.7 wt.%, titanium oxide 5.00 wt.%, mica titanium 3.00 wt.%, and a flavor 2.30 wt.% were well mixed by a stirrer to obtain an aimed composition.

### [Example 3]

Rosin 10.0 wt. %, Laccoat 50 EDS 20.0 wt. %, absolute ethanol 59.7 wt.%, titanium oxide 5.00 wt.%, mica titanium 3.00 wt.%, and a flavor 2.30 wt.% were well mixed by a stirrer to obtain an aimed composition.

### [Example 4]

Rosin 15.0 wt . %, Laccoat 50 EDS 20.0 wt. %, absolute ethanol 47.7 wt.%, titanium oxide-Yukaformer ethanol solution 15.0 wt. %, and a flavor 2.30 wt.% were well mixed by a stirrer to obtain an aimed composition. The ratio of titanium oxide to the entire composition is 1.5 wt.%.

### [Example 5]

Rosin 20.0 wt. %, Laccoat 50 EDS 20.0 wt.%, absolute ethanol 32. 7 wt. %, titanium oxide-Yukaformer ethanol solution 25 . 0 wt . %, and a flavor 2.30 wt.% were well mixed by a stirrer to obtain an aimed composition. The ratio of titanium oxide to the entire composition is 2.5 wt.%.

### [Example 6]

Rosin 15.0 wt.%, Laccoat 50 EDS 20.0 wt.%, absolute ethanol 49. 7 wt. %, titanium oxide-Yukaformer ethanol solution 5. 00 wt.%, titanium oxide 4.00 wt. %, mica titanium 3. 00 wt. %, a flavor 2.30 wt. %, and rosemary extract 1.00 wt.% were well mixed by a stirrer to obtain an aimed composition. The ratio of titanium oxide to the entire composition is 0.5 wt.%.

### [Example 7]

Rosin 5.00 wt.%, Laccoat 50 EDS 20.0 wt. %, absolute ethanol 64.4 wt.%, titanium oxide 3.80 wt.%, mica titanium 4.50 wt.%, and a flavor 2.30 wt.% were well mixed by a stirrer to obtain an aimed composition.

### [Example 8]

Rosin 5.00 wt.%, Laccoat 50 EDS 20.0 wt. %, absolute ethanol 63.2 wt.%, titanium oxide 3.80 wt.%, mica titanium 4.50 wt.%, HPC M (hydroxypropyl cellulose; manufactured by Nippon Soda Co., Ltd.) 0. 200 wt. %, a flavor 2. 30 wt. %, and Rome chamomile extract 1.00 wt.% were well mixed by a stirrer to obtain an aimed composition.

### [Example 9]

Rosin 5.00 wt. %, Laccoat 50 EDS 20.0 wt. %, absolute ethanol 63.0 wt.%, titanium oxide 2.00 wt.%, mica titanium 7.00 wt.%, sodium monofluorophosphate 0.700 wt.%, and a flavor 2.30 wt.% were well mixed by a stirrer to obtain an aimed composition.

### [Example 10]

Rosin 15.0 wt. %, Laccoat 50 EDS 20. 0 wt. %, absolute ethanol 39.75 wt.%, titanium oxide-Yukaformer solution 25.0 wt.%, and an aqueous 1.00% Red Color No. 106 solution 0.250 wt.% were well mixed by a stirrer to obtain an aimed composition. As the example, the composition added with a dye can be used for a tooth paint. The ratio of titanium oxide to the entire composition is 2.5 wt.%.

### [Comparative Example 1]

Rosin 40.0 wt.%, absolute ethanol 47.2 wt.%, titaniumoxide 2.00 wt.%, mica titanium 8.50 wt.%, and a flavor 2.30 wt.% were well mixed by a stirrer to obtain an aimed composition.

### [Comparative Example 2]

Rosin 20. 0 wt. %, absolute ethanol 69.7wt. %, titanium oxide 5.00 wt.%, mica titanium 3.00 wt.%, and a flavor 2.30 wt.% were well mixed by a stirrer to obtain an aimed composition.

### [Comparative Example 3]

Laccoat 50 EDS 50.0 wt.%, absolute ethanol 42.7 wt.%, titanium oxide 3. 00 wt.%, mica titanium 2.00 wt.%, and a flavor 2.30 wt.% were well mixed by a stirrer to obtain an aimed composition.

### [Comparative Example 4]

Laccoat 50 EDS 20.0 wt.%, absolute ethanol 69.4 wt.%, titanium oxide 3.80 wt.%, mica titanium 4. 50 wt.%, and a flavor 2.30 wt.% were well mixed by a stirrer to obtain an aimed composition.

### [Comparative Example 5]

Rosin 25.0 wt.%, absolute ethanol 47.7 wt.%, titanium oxide-Yukaformer ethanol solution 25.0 wt.%, and a flavor 2.30 wt. % were well mixed by a stirrer to obtain an aimed composition. The ratio of titanium oxide to the entire composition is 2.5 wt.%.

### [Comparative Example 6]

Laccoat 50 EDS 50.0 wt.%, absolute ethanol 22.7 wt.%, titanium oxide-Yukaformer ethanol solution 25.0 wt.%, and a flavor 2.30 wt. % were well mixed by a stirrer to obtain an aimed composition. The ratio of titanium oxide to the entire composition is 2.5 wt.%.

### (Test 1: Drying time test)

Each composition was applied to six front teeth of upper jaws of five examinees each one time without being overcoated in the state the teeth were kept being exposed and the time taken for the composition to be dried was measured. After the mouth was closed and it was confirmed that the formed coat was not removed and kept as it was even if the coat was touched with the tongue and measurement was repeated five times for every 15 second and the average time was calculated. The results of five examinees were averaged to measure the drying time.
The results are shown in Table 1.

### (Test 2: Test for luster at the time of application and durable duration of the luster)

The compositions which took 180 seconds or more to be dried in Test 1 (Example 5, Comparative Example 1, Comparative Example 5, and Comparative Example 6) were not suitable for practical application and they were eliminated and each of the remaining tooth coating compositions was applied to eight teeth of upper jaws of ten examinees and the luster was observed immediately after application and 2 hours and 4 hours after application without allowing the examinees to take meal (allowing them to take beverages) in the state the coat was dried by exposing the teeth.
The luster was evaluated according to the following four-grade standard.
Luster just like natural teeth: 3
Slightly decreased luster: 2
A little luster: 1
No luster: 0
The average values are shown in Table 1.

### (Test 3: Test of retention time of applied coat)

After ten examinees brushed teeth after breakfast, each tooth coating composition was applied to eight front teeth of upper jaws of them and the examinees were allowed not to take any thing for 30 minutes thereafter and asked to live normally as usual without brushing their teeth for ten hours. While they conduct a normal life without any diet and, the state of the formed coats were observed every 3 hours.
The evaluation was done according to the following four-grade standard.
No separation: 3
Separation only the tip end: 2
One third separated: 1
More than one third separated: 0
The average values after the respective hours are shown in Table 2.

### (Test 4: Test of removal easiness of used coat)

In Test 3, tooth brushing by normal brushing with a tooth brush was carried out after 12 hours (no tooth paste was used) and how much the coat was removed was observed.
The evaluation was done according to the following four-grade standard.
Completely removed: 3
Slightly remaining: 2
One third remaining: 1
More than one third remaining: 0
The average values of ten examinees were shown in Table 2.

### (Test 5: Test for hiding power)

The tooth coating compositions of Examples and Comparative Examples were applied only once to color models of artificial teeth VITAPAN Classical Shade Guide (VITA Inc.) A2, A3, and A4 (yellowish color models, the color becomes deeper as the number is increased more) and C2, C3, and C4 (blackish color models, the color becomes deeper as the number is increased more) to evaluate the hiding power.
Example 2: with respect to A2, A3, and A4, bright and white coats hiding the color of the artificial teeth thereunder were obtained: and
with respect to C2, C3, and C4, bright and white coats hiding the color of the artificial teeth thereunder were obtained.
Example 3: with respect to A2, A3, andA4, bright and white coats hiding the color of the artificial teeth thereunder were obtained: and
with respect to C2, C3, and C4, bright and white coats hiding the color of the artificial teeth thereunder were obtained.
Example 6: with respect to A2, A3, and A4, bright and white coats hiding the color of the artificial teeth thereunder were obtained: and
with respect to C2, C3, and C4, bright and white coats hiding the color of the artificial teeth thereunder were obtained.
Comparative Example 2: with respect to A2, A3, and A4, bright and white coats hiding the color of the artificial teeth thereunder were obtained: and
with respect to C2, C3, and C4, bright and white coats hiding the color of the artificial teeth thereunder were obtained.
Comparative Example 3: with respect to A2, A3, and A4, bright and white coats hiding the color of the artificial teeth thereunder were obtained: and
with respect to C2, C3, and C4, bright and white coats hiding the color of the artificial teeth thereunder were obtained.
Example 1: with respect to A2 and A3, bright and white coats hiding the color of the artificial teeth thereunder were obtained: and
with respect to A4, the color became close to that of A2 without being coated with the tooth coating composition and seemed to be more blackish than the color of A2 being coated with the tooth coating composition: and
with respect to C2 and C3, bright and white coats hiding the color of the artificial teeth thereunder were obtained: and
with respect to C4, the color became close to that of C2 without being coated with the tooth coating composition and seemed to be more blackish than the color of C2 being coated with the tooth coating composition.
Example 4: with respect to A2 and A3, bright and white coats hiding the color of the artificial teeth thereunder were obtained: and
with respect to A4, the color became close to that of A2 without being coated with the tooth coating composition and seemed to be more blackish than the color of A2 being coated with the tooth coating composition: and
with respect to C2 and C3, bright and white coats hiding the color of the artificial teeth thereunder were obtained: and
with respect to C4, the color became close to that of C2 without being coated with the tooth coating composition and seemed to be more blackish than the color of C2 being coated with the tooth coating composition.
Comparative Example 4: with respect to A2 and A3, bright and white coats hiding the color of the artificial teeth thereunder were obtained: and
with respect to A4, the color became close to that of A2 without being coated with the tooth coating composition and seemed to be more blackish than the color of A2 being coated with the tooth coating composition: and
with respect to C2 and C3, bright and white coats hiding the color of the artificial teeth thereunder were obtained: and
with respect to C4, the color became close to that of C2 without being coated with the tooth coating composition and seemed to be more blackish than the color of C2 being coated with the tooth coating composition.

**Table 1 (Test 1: Drying time test) (Test 2: Test for luster and duration of the luster)**

| | Average drying time (second) | Luster | | |
|---|---|---|---|---|
| | | Immediately after application | 2 hours later | 4 hours later |
| Example 1 | 60 | 3.0 | 2 . 6 | 1.4 |
| Example 2 | 45 | 3.0 | 2.6 | 1.4 |

| | | | | |
|---|---|---|---|---|
| Example 3 | 30 | 2 . 6 | 1.2 | 0.6 |
| Example 4 | 60 | 3.0 | 2. 6 | 1.2 |
| Example 5 | 180 | - | - | - |
| Example 6 | 45 | 1.6 | 0.8 | 0.4 |
| Example 7 | 30 | 1. 2 | 1. 0 | 0.4 |
| Example 8 | 45 | 1.2 | 1.0 | 0.4 |
| Comparative Example 1 | 255 | - | - | - |
| Comparative Example 2 | 150 | 2.4 | 1.8 | 0.8 |
| Comparative Example 3 | 150 | 3.0 | 0.8 | 0 |
| Comparative Example 4 | 20 | 1.2 | 0.8 | 0 |
| Comparative Example 5 | 210 | - | - | - |
| Comparative Example 6 | 180 | - | - | - |

**Table 2 (Test 3: Retention time of applied coat; Test 4: Removal easiness of used coat)**

| | 3 hours later | 6 hours later | 9 hours later | 12 hours later | removal property |
|---|---|---|---|---|---|
| Example 1 | 3.0 | 2.8 | 2.6 | 2.6 | 2.5 |
| Example 2 | 3.0 | 2.8 | 2.8 | 2.7 | 2.5 |
| Example 3 | 3.0 | 2.7 | 2.7 | 2.6 | 2.7 |
| Example 4 | 3.0 | 2.8 | 2. 8 | 2.5 | 2.7 |
| Example 6 | 3.0 | 2.6 | 2.6 | 2.6 | 2.7 |
| Example 7 | 3.0 | 2.8 | 2.8 | 2.6 | 3.0 |
| Example 8 | 3.0 | 2.8 | 2.7 | 2.5 | 3.0 |
| Comparative Example 2 | 2.8 | 1.8 | 1.6 | 1.4 | 3.0 |
| Comparative Example 3 | 2.8 | 2.4 | 2.2 | 2.0 | 2.8 |
| Comparative Example 4 | 3.0 | 2.8 | 2.8 | 2.8 | 3.0 |

The results of the above-mentioned comparative tests made the following clear.
(1) In the case shellac or rosin is used alone and the coat is tried to be thick by increasing the concentration, it took long for drying (Comparative Example 1, Comparative Example 3, Comparative Example 5, and Comparative Example 6). The coats were made thick by increasing the resin concentration without prolonging the drying time in the case both were mixed at proper ratio (Examples 1 to 4 and Examples 6 to 8).

(2) If the concentration was increased, particularly the ratio of shellac was increased, the luster was lost and the coats became opaque with the passage of time to worsen the aesthetic property (Comparative Example 3 and Comparative Example 4). In this case also, mixing of shellac and rosin could rather efficiently suppress the coats from becoming opaque (Examples 1 to 4 and Examples 6 to 8).

(3) Although the drying time took a slightly longer time for the tooth coating compositions in which the titanium-Yukaformer ethanol solution was added as a dispersant for a pigment, the tooth coating compositions gave higher luster than the tooth coating compositions in which the pigments were added directly (Example 1, Example 4, and Example 6). No deterioration of the durable duration of the applied coats owing to the addition of the dispersant (20 wt. % or lower) was observed. Further, since the pigment were evenly dispersed, the content of the pigments could be lowered and uneven dispersion was hardly caused and the viscosity was increased to make coating easy.

(4) From the test of hiding force, although the color of the applied teeth could not completely be hided since the ratio of the pigment was low in Example 1 and Example 4, it was found that the hiding power was heightened in Example 2, Example 3, and Example 6 in which the ratio of the pigment was high. Although the addition amount of the pigment was high in Comparative Example 4, the hiding force was low since the resin amount was low. It was found that although the content of the pigment scarcely affected the durable duration of applied coats, if the content was lowered, the tooth color-hiding power was deteriorated.

(5) The adhesion property was improved by increasing the resin concentration; nevertheless, the coats were found removable by normal tooth brushing with a brush, without requiring any special tool for removal.

### [Industrial Applicability]

The tooth coating composition of the invention can be used as a tooth coating agent for beauty and appearance or health, and also for a coloration paint for drawing a design on teeth for a purpose of arts and an adhesive for sticking seals made of paper and plastics, crushed stone with beautiful color, and powdered or fragmented luster reflecting agents to teeth for purposes of arts.

## Claims

1. A tooth coating composition containingshellac,rosin, and their solvent as the main components.

2. The tooth coating composition according to claim 1, wherein the content of the shellac is in a range of 1 wt.% to 30 wt.%, preferably in a range of 5 wt. % to 20 wt.%, in the entire composition and the content of the rosin is in a range of 1 wt.% to 30 wt.%, preferably in a range of 5 wt.% to 25 wt.%, in the entire composition.

3. The tooth coating composition according to claim 2, wherein the total content of the shellac and the rosin is in a range of 10 wt.% to 31 wt.% in the entire composition.

4. The tooth coating composition according to claim 1, wherein one or more of auxiliary components selected from a pigment, a pigment dispersant, a dye, an efficacious component having anti-dental caries function, an anti-bacterial agent, a tackifier, a luster reflecting material, a bleaching agent, and a flavor are added to the composition.

5. The tooth coating composition according to claim 4, wherein one or more of pigments selected from titanium oxide, natural pearl, mica titanium, iron oxide, a body extender pigment, and a synthetic coloring agent are added as the pigment.

6. The tooth coating composition according to claim 4, wherein the content of the pigment is in a range of 1 wt.% to 20 wt.%, in the entire composition.

7. The tooth coating composition according to claim 4, wherein
N-methacrylethyl-N,N-dimethylammonium-α-N-methylcarboxybeta ine-alkyl methacrylate copolymer is used as the dispersant of the pigment.

8. The tooth coating composition according to claim 4, wherein the content of the dispersant of the pigment is in a range of 1 wt.% to 20 wt.%, in the entire composition.

9. The tooth coating composition according to claim 4, wherein one or more of compound selected from sodium fluoride, sodium monofluorophosphate, and stannous fluoride are added as efficacious components having anti-caries function to the composition.

10. The tooth coating composition according to claim 4, wherein one or more of compounds selected from sodium azulenesulfonate, ε-aminocaproic acid, allantoin, allantoin chlorohydroxyaluminum, allantoin dihydroxyaluminum, epidihydrocholesterol, dihydrocholesterol, sodium chloride, glycyrrhizic acid, diammonium glycyrrhizinate, disodium glycyrrhizinate, trisodium glycyrrhizinate, dipotassium glycyrrhizinate, monoammonium glycyrrhizinate, β-glycyrrhetic acid, isopropylmethylphenol, cetylpyridinium chloride, decalinium chloride, benzalconium chloride, benzethonium chloride, alkyldiaminoethylglycine hydrochloride, chlorohexidine hydrochloride, triclosan, ascorbic acid, sodium ascorbate, pyridoxine hydrochloride, dl-α-tocopherol acetate, dl-α-tocopherol nicotinate, zeolite, sodium dihydrogen pyrophosphate, sodium pyrophosphate, sodium monohydrogen phosphate, trisodium phosphate, sodium polyphosphate, polyethylene glycol, polyvinyl pyrrolidone, lysozyme chloride, sodium copper chlorophyllin, hinokitiol, polyoxyethylene lauryl ether, sodium lauroyl sarcosinate, plant extract, and phytoncide are used as the anti-bacterial agent.

11. The tooth coating composition according to claim 4, wherein hydroxypropyl cellulose is used as the tackifier.

12. The tooth coating composition according to claim 4, wherein one or more of compounds selected from hydrogen peroxide, calcium peroxide, and urea peroxide are used as the bleaching agent.

13. A tooth coating composition to be used as an adhesive for an art material such as a seal, crushed stone, or luster reflecting material, wherein the composition contains shellac, rosin, and their solvent as main components.

14. The tooth coating composition according to claim 13, wherein the content of the shellac is in a range of 1 wt.% to 30 wt.% in the entire composition and the content of the rosin is in a range of 1 wt.% to 30 wt.% in the entire composition.
